# FASCICULE DE BREVET EUROPEEN

(11) **EP 1 040 353 B1**
(45) Date de publication et mention de la délivrance du brevet: **18.08.2004**
(21) Numéro de dépôt: 99952164.4
(22) Date de dépôt: 20.10.1999
(51) Int. Cl.: G01N 33/58, G01N 33/543

(54) **PROCEDE DE DEPISTAGE D'ANALYTE ET TROUSSE**
VERFAHREN ZUR BESTIMMUNG EINES ANALYTEN UND KIT DAFÜR
METHOD FOR DETECTING AN ANALYTE AND KIT

(30) Priorité: 21.10.1998 BE 9800754
(43) Date de publication de la demande: 04.10.2000
(73) Titulaire: D-TEK, 7000 Mons (BE)
(72) Inventeur: VIGNERON, Alain, Georges, André, B-7331 Baudour (BE); BOUVIER, Jacques, Albert, Georges, Ghislain, B-4550 Nandrin (BE)
(74) Mandataire: Claeys, Pierre
(86) Numéro de dépôt international: PCT/BE1999/000129
(87) Numéro de publication internationale: WO 2000/023805

(56) Documents cités:
- EP-A- 0 833 157
- DE-A- 19 731 465
- US-A- 5 356 782

## Description

La présente invention est relative à un procédé de dépistage d'au moins un analyte dans un échantillon de fluide biologique à analyser, comprenant :
- une mise en contact de l'échantillon de fluide biologique avec un ligand, qui est spécifique d'au moins un analyte à dépister et qui est fixé sur au moins une zone d'essai d'un support solide,
- une liaison entre ledit au moins un analyte à dépister et le ligand,
- un marquage du ligand spécifique et dudit au moins un analyte liés, par un traceur, et
- une détection du traceur de marquage sur le ligand spécifique et ledit au moins un analyte liés, avec détermination d'une intensité de signal de marquage,
- une mise en contact de l'échantillon de fluide biologique avec un ligand non spécifique dudit au moins un analyte, ledit ligand non spécifique étant fixé sur une zone de contrôle du support solide et étant capable de rendre compte d'interactions non spécifiques engendrées par l'échantillon,
- une adsorption et/ou un accrochage de facteurs d'interactions non spécifiques de l'échantillon sur le ligand non spécifique,
- un marquage du ligand non spécifique et des facteurs d'interactions liés, par un traceur,
- une détection du traceur de marquage sur le ligand non spécifique et les facteurs d'interactions liés, avec détermination d'une intensité de signal de marquage,

On connaît depuis très longtemps des procédés de dépistage (voir par exemple les US-A-4376110, US-A-4703017 et US-A-5028535).

Ce type de dépistage est communément appelé un test "immunodot".

Par support solide il faut entendre dans la présente invention un substrat de préférence en forme de feuille ou membrane, qui peut se présenter par exemple sous la forme d'une carte, d'un ruban ou d'une tigette. On utilise fréquemment une membrane cellulosique, notamment en nitrocellulose ou en esters de cellulose mixtes d'acide nitrique et d'autres acides. Il est évident que des supports solides ayant une autre forme et/ou une autre nature peuvent être envisagés pour la mise en oeuvre du procédé.

Par ligand spécifique, on peut entendre toute molécule pour laquelle il existe une molécule complémentaire spécifique, c'est-à-dire l'analyte à détecter, et qui soit capable de se fixer sur le support solide. Cette dernière fixation peut se faire au travers d'une série d'interactions différentes, telles que des liaisons ioniques, des liaisons covalentes, des interactions hydrophobes, etc...

Par analyte, on comprend toute molécule dont la détection et la mesure de la concentration sont à réaliser et pour laquelle il existe une molécule complémentaire spécifique. Le ligand spécifique et l'analyte peuvent par exemple être stéréocomplémentaires ce qui permet la fixation de l'analyte sur le support solide par l'intermédiaire du ligand facilement accessible à la surface du support solide. On peut envisager, comme analyte, des antigènes, des anticorps, des protéines, des haptènes, des acides nucléiques, etc... Les termes de ligand spécifique et d'analyte peuvent d'ailleurs s'appliquer à un même type de molécule selon la conception de l'essai.

Par traceur, on peut entendre une molécule spécifique qui est complémentaire de l'analyte et à laquelle est couplé un système permettant sa détection, par exemple une enzyme, un marqueur radioactif, un marqueur fluorescent ou luminescent, une particule colorée (polymère, particules d'or ou de latex), etc..

Les essais faisant appel à un procédé de dépistage tel que décrit ci-dessus peuvent être utilisés pour la détection d'analytes spécifiques dans des fluides biologiques, tels que du sérum, du sang, de l'urine, etc... Des essais immunologiques peuvent être envisagés. Ces essais peuvent être utilisés pour des mesures semi-quantitatives ou quantitatives d'un ou de plusieurs analytes cibles, en une seule opération, avec ou sans utilisation d'une instrumentation spécifique pour la détection d'un signal. Dans ce type de test, l'intensité du signal de marquage est directement proportionnelle à la concentration de l'analyte à détecter dans l'échantillon.

Plusieurs types d'essais immunologiques sur phase solide ont été décrits, dans lesquels la présence ou l'absence d'un analyte dans un échantillon peut être déterminée par une interprétation visuelle des résultats (voir par exemple le US-A-4703017).

La réponse à ces tests se limite cependant à une simple alternative (positif/négatif) et une interprétation semi-quantitative ou quantitative implique d'effectuer en parallèle des essais séparés avec des standards (contrôles) dont la concentration en analyte est connue. La relation entre la réponse observée et la concentration de l'analyte dans l'échantillon peut alors être établie par comparaison avec la ou les références.

On connaît également des procédés de dépistage incluant des systèmes de calibration interne qui évitent l'utilisation de plusieurs essais pour évaluer la concentration d'un analyte d'un seul échantillon (voir par exemple US-A-5028535).

Dans toute application visant à mesurer une interaction spécifique entre deux ou plusieurs macromolécules organiques, le problème majeur consiste en l'existence d'un bruit de fond résultant d'interférences non spécifiques entre les molécules en présence. Chaque échantillon à analyser contient des facteurs d'interactions non spécifiques qui sont par exemple des molécules interférentes, distinctes de l'analyte à détecter, qui, par leurs propriétés chimiques intrinsèques, possèdent la faculté de se lier non spécifiquement au ligand spécifique (par exemple par accrochage non stéréocomplémentaire ou adsorption) et spécifiquement ou non au traceur. Par exemple, dans un essai de dépistage d'anticorps spécifiques, les immunoglobulines autres que les anticorps spécifiques dirigés contre l'antigène utilisé comme ligand spécifique peuvent représenter un facteur d'interactions non spécifiques.

Par autre facteur d'interactions non spécifiques, on doit aussi comprendre une liaison non spécifique de l'analyte à détecter avec au moins un ligand non spécifique, c'est-à-dire, par accrochage non stéréocomplémentaire ou adsorption. Cela signifie que l'analyte à détecter peut lui aussi participer au bruit de fond à déterminer, au contraire de l'enseignement des brevets US 5,356,782 et EP 0833157.

Par bruit de fond, on doit donc comprendre l'intensité du signal non spécifique résultant d'un ensemble de facteurs d'interactions moléculaires non spécifiques, liés à la nature de l'échantillon (par exemple présence et concentration variables de substances susceptibles de se lier non spécifiquement au ligand spécifique de l'analyte et au traceur, pH, concentration en sels, teneur en lipides variables favorisant les adsorptions intermoléculaires non spécifiques) ou aux conditions dans lesquelles le test est effectué (par exemple température, temps d'incubation, concentrations de l'échantillon et du traceur).

Par potentiel de bruit de fond, on doit par contre comprendre l'intensité maximale de signal non spécifique susceptible d'être engendrée sur une zone d'essai par un échantillon donné, dans des conditions de test données. Par définition, chaque échantillon peut donc être caractérisé par un potentiel de bruit de fond qui lui est propre et intrinsèquement lié.

La mesure du potentiel de bruit de fond donne la valeur seuil de l'intensité de signal, au-dessus de laquelle un échantillon est considéré comme positif et en dessous de laquelle il est considéré comme négatif.

Il va de soi que, chaque échantillon possédant ses propres caractéristiques physico-chimiques, chaque échantillon est susceptible de générer un bruit de fond qui lui est propre et possède intrinsèquement un potentiel de bruit de fond qui lui est propre et peut donc se voir attribuer une valeur seuil qui lui est propre.

Les performances d'un test dépendent de la précision avec laquelle la valeur seuil est mesurée; il importe donc de pouvoir estimer le potentiel de bruit de fond d'un échantillon de manière à obtenir une valeur idéalement corrigée de l'interaction spécifique. Or, dans la plupart des tests actuels de dépistage d'un analyte dans un échantillon de fluide biologique, on détermine de manière générique une valeur seuil unique applicable à l'ensemble des échantillons testés sans tenir compte de la variabilité du bruit de fond lié à chaque échantillon.

Dans un test ELISA par exemple, la détermination d'une valeur seuil (cut-off) de l'intensité du signal, au-dessus de laquelle un échantillon testé est considéré comme positif et en dessous de laquelle il est considéré comme négatif, est effectuée indirectement grâce à au moins un contrôle dont la concentration en analyte est connue, testé en parallèle sur au moins une zone d'essai équivalente mais distincte (par exemple au moins une cuvette voisine dans un test ELISA) avec le ou les échantillon(s) à analyser. La valeur de seuil est généralement définie par rapport aux valeurs données par une population statistiquement significative d'échantillons considérés comme négatifs pour l'analyte à dépister : un traitement statistique de la distribution de ces valeurs permet de calculer la valeur seuil à utiliser dans l'interprétation des résultats en fonction des performances souhaitées (spécificité et sensibilité du test) (v. par exemple, M.P. BOUNAUD, et J-F. MORIN, Evaluation analytique d'un nouvel immunodosage, Atelier n° 13 du 10e Colloque CORATA, 1993). Bien que ce type d'approche tienne compte de la variabilité de la valeur seuil en fonction des conditions dans lesquelles le test est effectué (les contrôles étant toujours testés en parallèle avec les échantillons dans les mêmes conditions), la variabilité du bruit de fond engendré par les différents échantillons eux-mêmes n'est pas prise en considération (les contrôles ne pouvant pas rendre compte de la présence au sein de chaque échantillon d'un plus ou moins grand nombre de facteurs d'interactions non spécifiques à une plus ou moins grande concentration). Ceci est susceptible de limiter sévèrement la sensibilité du test et de mener à des interprétations faussement négatives pour certains échantillons, particulièrement dans le cas d'une combinaison d'un test hautement spécifique (pour lequel la valeur seuil est nécessairement proche du signal maximal rencontré dans une population négative) et d'un échantillon faiblement positif qui posséderait à fortiori un faible potentiel de bruit de fond.

Le document DE 197 31 465 A1 non publié à la date de priorité de la présente demande de brevet décrit un système de contrôle(s) interne(s) permettant de mesurer indépendamment la valeur d'une ou de plusieurs interactions non spécifiques engendrées par un ou plusieurs facteurs d'interférence propres à chaque échantillon. Néanmoins, la mise en oeuvre de tels contrôles internes implique que les différents facteurs d'interférence soient connus et définis et qu'à chaque facteur d'interférence défini soit associé un contrôle spécifique distinct. Cette approche permet de mesurer de manière précise l'intensité du signal spécifique par rapport à chacune des interactions non spécifiques qui ont été définies; par contre, le système ne permet pas d'obtenir une évaluation globale, par l'intermédiaire d'un seul et unique contrôle, du bruit de fond global potentiellement engendré par l'ensemble des facteurs d'interférences, connus ou non, présents dans l'échantillon.

Aucun document antérieur n'apporte de solutions à ce problème.

Or, sans possibilité d'évaluation d'un seuil réactionnel propre à l'échantillon analysé, l'interprétation des résultats de la plupart des essais immunodot se résume aux deux possibilités suivantes : l'essai est positif si une coloration est visible au niveau de la zone d'essai, et l'essai est négatif en cas d'absence de coloration. Si le système fonctionne effectivement dans les cas où une coloration forte ou une totale absence de coloration est évidente, il est manifeste que l'interprétation des résultats est soumise à la subjectivité du lecteur dans les cas où seule une trace de coloration est visible. Cette trace peut être le reflet d'une interaction spécifique antigène - anticorps, mais aussi simplement un bruit de fond.

La présente invention a pour but de résoudre ces problèmes en mettant au point un procédé du type décrit au début, qui permette d'établir un auto-étalonnage intégré à l'essai avec pour chaque échantillon un seuil de réactivité distinct, qui peut être variable en fonction des caractéristiques physico-chimiques de l'échantillon lui-même et des conditions dans lesquelles l'essai est effectué.

On a résolu le problème posé par un procédé tel que décrit au début, ce procédé étant caractérisé en ce que :
au moins un ligand non spécifique est fixé sur une zone de contrôle dite de seuil, en une concentration telle que l'intensité de signal de marquage déterminée là pour un échantillon seuil de fluide biologique est égale à celle de cet échantillon seuil sur la zone d'essai,
ledit au moins un ligand non spécifique de la zone de contrôle de seuil étant capable de rendre compte d'un potentiel de bruit de fond engendré sur au moins une zone d'essai par un ensemble de facteurs d'interactions non spécifiques, liés à la nature de l'échantillon lui-même et aux conditions dans lesquelles le test est effectué,
et en ce que le procédé comprend une comparaison entre l'intensité du signal de marquage de la zone d'essai et celle de la zone de contrôle de seuil obtenues avec l'échantillon à analyser, celui-ci étant à considérer comme positif lorsque l'intensité du signal de marquage de la zone d'essai est supérieure à celle de la zone de contrôle de seuil.

L'échantillon de fluide biologique appelé échantillon seuil est un des échantillons d'une population statistiquement significative qui, lors d'une analyse de celle-ci par des techniques de référence standards, a été diagnostiqué comme négatif, mais qui dans son application à une zone d'essai d'un procédé de dépistage immunodot donnait une intensité de signal de marquage correspondant à une valeur seuil telle qu'elle peut être calculée statistiquement ou estimée (selon que la lecture se fait respectivement avec ou sans appareil de mesure) par rapport à la distribution des valeurs données par l'ensemble des échantillons négatifs de cette population. Dans le cas où aucun des échantillons négatifs de la population ne donne précisément l'intensité seuil de signal telle que calculée ou estimée, l'échantillon seuil utilisé pour la calibration peut être un échantillon dont l'intensité de signal approche la valeur seuil avec suffisamment d'exactitude, ou encore un calibrateur artificiellement construit (généralement un sérum positif dilué) donnant l'intensité de signal désirée et qui est dès lors considéré comme représentatif de l'échantillon seuil.

L'intensité de signal de marquage obtenue sur la zone de contrôle de seuil avec cet échantillon seuil est donc uniquement due à un bruit de fond, que celui-ci résulte d'interférences extérieures à l'analyte ou de liaisons non spécifiques de l'analyte sur le ligand non spécifique. Les expériences ont montré que, lors de l'application du procédé de dépistage suivant l'invention, l'intensité de signal de marquage des échantillons testés sur la zone de contrôle de seuil variait d'échantillon à échantillon et représentait donc une valeur de bruit de fond propre à chaque échantillon. En outre, il est remarquable de constater que pour la plupart des échantillons négatifs, l'intensité du signal de marquage sur la zone de contrôle de seuil est supérieure à celle obtenue sur la zone d'essai. Le système rend donc bien compte d'un potentiel de bruit de fond susceptible d'être généré par un échantillon sur une zone d'essai, et non du bruit de fond réellement engendré par cet échantillon sur ladite zone d'essai. Par comparaison avec l'intensité de signal de marquage obtenue sur la zone d'essai, on obtient une détermination directe et auto-corrigée du caractère positif ou négatif de l'échantillon, selon que l'intensité sur la zone d'essai est supérieure ou inférieure à l'intensité sur la zone de contrôle de seuil.

Il faut remarquer que par le procédé suivant l'invention le ligand non spécifique de la zone de contrôle de seuil rend compte d'un potentiel de bruit de fond généré par des interactions non spécifiques engendrées par l'échantillon, interactions qui ne sont ni nécessairement connues ni définies. Un tel dépistage comporte par ailleurs une seule et unique zone de contrôle de seuil par échantillon à analyser.

Suivant une forme de réalisation avantageuse de l'invention, le procédé comprend
- une mise en contact de l'échantillon de fluide biologique à analyser avec au moins un ligand qui est non spécifique dudit au moins un analyte et qui est fixé sur une zone de contrôle dite de réaction du support solide,
- une adsorption et/ou un accrochage de facteurs d'interactions non spécifiques de l'échantillon sur ledit au moins un ligand non spécifique de la zone de contrôle de réaction,
- un marquage dudit au moins un ligand non spécifique de la zone de contrôle de réaction et des facteurs d'interaction liés, par un traceur,
- une détection du traceur dé marquage sur ledit au moins un ligand non spécifique de la zone de contrôle de réaction et les facteurs d'interaction liés, avec détermination d'une intensité de signal de marquage,
- ledit au moins un ligand non spécifique de la zone de contrôle de réaction étant fixé sur celle-ci de manière que l'intensité de signal de marquage déterminée soit détectable lorsque le procédé de dépistage a été appliqué d'une manière conforme.

Le procédé permet de s'assurer que la séquence des étapes du test a été respectée et que les différents composants mis en oeuvre sont fonctionnels. L'essai n'est pas valable si une intensité de signal de marquage élevée sur la zone de contrôle de réaction n'apparaît pas. Pour que ledit au moins un ligand non spécifique de la zone de contrôle permette une détection efficace, on peut jouer sur sa concentration ou sur sa nature.

Les expériences ont montré que, comme pour les zones de contrôle de seuil, les zones de contrôle de réaction présentent des intensités de signal de marquage qui sont variables, c'est-à-dire propres à l'échantillon analysé, et cela parce que toutes deux incluent une intensité de signal due au bruit de fond propre à l'échantillon.

Suivant une forme de réalisation perfectionnée de l'invention, le procédé comprend
- une mise en contact de l'échantillon de fluide biologique avec au moins un ligand supplémentaire, qui est spécifique d'au moins un analyte supplémentaire à dépister et qui est fixé sur au moins une zone d'essai supplémentaire du support solide,
- une liaison entre ledit au moins analyte supplémentaire et son ligand spécifique,
- un marquage dudit au moins un ligand supplémentaire et dudit au moins un analyte supplémentaire liés, par un traceur, et
- une détection du traceur de marquage sur ledit au moins un ligand supplémentaire et ledit au moins un analyte supplémentaire liés, avec détermination d'une intensité de signal de marquage,
- chaque ligand spécifique supplémentaire étant fixé dans sa zone d'essai supplémentaire en une concentration telle que l'intensité de signal de marquage déterminée là pour un échantillon seuil pour l'analyte supplémentaire correspondant est égale ou inférieure à l'intensité de signal de marquage obtenue dans ladite zone de contrôle de seuil, et
- une comparaison entre l'intensité du signal de marquage de chaque zone d'essai supplémentaire et celle de la zone de contrôle de seuil, obtenues avec l'échantillon à analyser, celui-ci étant à considérer comme positif pour un analyte supplémentaire lorsque l'intensité du signal de marquage de la zone d'essai supplémentaire correspondant à cet analyte supplémentaire est supérieure à celle de la zone de contrôle de seuil.

Il est ainsi possible d'effectuer la détection de plusieurs analytes différents en se référant à une seule zone de contrôle de seuil pour chaque échantillon.

Ledit au moins un ligand non spécifique fixé sur la zone de contrôle de seuil peut être identique audit au moins un ligand non spécifique fixé sur la zone de contrôle de réaction, mais à une concentration plus faible. Il peut aussi comprendre des substances différentes.

Ledit au moins un ligand non spécifique de contrôle de seuil peut par exemple comprendre :
- un anticorps, y compris les anticorps monoclonaux, dirigé ou non contre un ou plusieurs antigènes contenus dans l'échantillon, par exemple contre des immunoglobulines d'espèce animale ou humaine,
- un antigène, dirigé ou non contre un ou plusieurs anticorps contenus dans l'échantillon,
- une protéine, comme la protéine A, la protéine G ou la protéine L capable d'accrocher le fragment Fc de certaines immunoglobulines,
- une substance naturelle, comme de l'albumine de sérum de boeuf (BSA) ou les protéines du lait de vache (la caséine par exemple), capable d'accrocher non spécifiquement certains polypeptides par le biais d'interactions protéine-protéine non stéréocomplémentaires,
- une séquence synthétique ou non d'acides aminés, comme par exemple un peptide synthétique, un haptène, de la polylysine, qui est capable d'interactions non spécifiques avec certaines molécules organiques,
- une séquence nucléotidique synthétique ou naturelle, comme par exemple de l'ADN (simple brin ou double brin) ou de l'ARN.

La sélection du ligand ou du mélange de ligands de contrôle de seuil idéal dépend généralement, mais de manière non limitative, du type d'essai, donc de la nature des facteurs d'interactions non spécifiques qui sont susceptibles de se présenter dans ce cas particulier. La concentration idéale du ligand ou du mélange de ligands à appliquer sur la zone de contrôle de seuil est quant à elle définie exclusivement au travers du processus de calibration tel que décrit précédemment.

La présente invention concerne également une trousse de dépistage d'analyte.

Cette trousse comprend :
- un support solide,
- un ligand qui est spécifique d'au moins un analyte à dépister et est fixé sur au moins une zone d'essai du support solide, et qui, après mise en contact avec l'échantillon à analyser, se lie à ledit au moins un analyte à dépister, en présence de celui-ci,
- un traceur capable de marquer le ligand spécifique et ledit au moins un analyte liés, en permettant sa détection avec détermination d'une intensité de signal de marquage,
- un ligand qui est non spécifique dudit au moins un analyte et est fixé sur une zone de contrôle du support solide, et qui, après mise en contact avec l'échantillon à analyser, permet une adsorption et/ou un accrochage sur lui de facteurs d'interactions non spécifiques engendrés par l'échantillon, et
- un traceur capable de marquer le ligand non spécifique et les facteurs d'interactions liés, en permettant sa détection avec détermination d'une intensité de signal de marquage,
cette trousse comportant en outre, suivant l'invention, au moins un ligand non spécifique fixé sur une zone de contrôle dite de seuil du support solide en une concentration telle que l'intensité de signal de marquage, déterminée là pour un échantillon seuil de fluide biologique est égale à celle de cet échantillon seuil sur la zone d'essai, ledit au moins un ligand non spécifique de la zone de contrôle de seuil étant capable de rendre compte d'un potentiel de bruit de fond engendré sur au moins une zone d'essai par un ensemble de facteurs d'interactions non spécifiques, liés à la nature de l'échantillon lui-même et aux conditions dans lesquelles le test est effectué,
l'échantillon à analyser étant à considérer comme positif lorsque l'intensité du signal de marquage de la zone d'essai est supérieure à celle de la zone de contrôle de seuil.

Des détails particuliers concernant le procédé de dépistage et la trousse suivant l'invention sont indiqués dans les revendications données ci-après.

D'autres détails et particularités de l'invention ressortiront de la description donnée ci-dessous, avec référence aux dessins annexés.

### 1. Détermination de la concentration du ligand spécifique sur la zone d'essai.

Le ligand spécifique pour l'analyte à détecter est dilué en série généralement dans une solution saline physiologique à dix concentrations variant généralement de 1 à 0,1 µg par millilitre.

Chaque dilution est appliquée sous forme de gouttelettes de 1 microlitre sur différentes portions d'une membrane généralement de type nitrocellulosique, l'ensemble constituant une bandelette.

Les bandelettes ainsi préparées sont testées sur une population d'échantillons positifs et négatifs statistiquement significative, selon une procédure courante pour l'homme de métier.

La concentration idéale de ligand spécifique (soit la concentration X) est celle permettant la meilleure discrimination visuelle entre l'échantillon positif donnant l'intensité la plus faible et l'échantillon négatif donnant l'intensité la plus forte.

### 2. Détermination de la concentration dudit au moins un ligand non spécifique sur la zone de contrôle de seuil.

Le ligand spécifique est appliqué, à la concentration déterminée ci-dessus (soit la concentration X), sur une portion de membrane, et cette zone d'essai sert ici de référence.

Ledit au moins un ligand non spécifique est dilué en série et appliqué, comme décrit en 1, sur le reste des portions de membrane libres de la bandelette.

Les bandelettes sont testées sur la même population d'échantillons.

Un échantillon négatif, donnant sur la zone d'essai (qui contient le ligand spécifique à la concentration X) une intensité seuil, telle qu'elle peut être calculée ou estimée selon une procédure courante pour l'homme de métier, est utilisé pour calibrer le seuil : la concentration idéale (soit la concentration Y) du ligand non spécifique est celle donnant, sur la zone de contrôle de seuil, une intensité de coloration identique à celle observée sur la zone d'essai.

L'ensemble des autres échantillons permet de valider le seuil ainsi déterminé : tous les échantillons positifs donnent une intensité supérieure au seuil et tous les échantillons négatifs donnent une intensité inférieure au seuil nonobstant les limites de spécificité et de sensibilité du test telles qu'imposées par la procédure de calcul ou d'estimation de l'intensité seuil. L'intensité du seuil déterminé varie elle-même en fonction des caractéristiques physio-chimiques de chaque échantillon.

### 3. Essai à plusieurs analytes.

Le développement d'un test à plusieurs analytes implique de suivre dans un premier temps la procédure décrite dans 1 et 2 ci-dessus pour un seul de ces analytes. On détermine alors la concentration idéale dudit au moins un ligand non spécifique (soit la concentration Y) pour cet analyte.

Ledit au moins un ligand non spécifique est appliqué, à la concentration déterminée ci-dessus (soit la concentration Y), sur une portion de membrane, et cette zone sert ici de référence.

Les autres ligands spécifiques des autres analytes sont chacun dilués en série et appliqués, comme décrit sous 1, sur le reste des portions de membrane libres de la bandelette. Les bandelettes sont testées sur une population d'échantillons statistiquement significative pour les autres analytes précités.

Conformément à la procédure décrite en 2, on détermine alors la concentration idéale de chacun des autres ligands spécifiques des autres analytes par rapport audit au moins un ligand non spécifique à la concentration Y, c'est-à-dire en fonction d'un seul et même seuil.

Accessoirement, il est généralement possible d'inclure au test un contrôle de réaction en déposant sur une portion de membrane une concentration dite saturante dudit au moins un ligand non spécifique de contrôle de seuil (concentration généralement 50 à 100 fois supérieure à la concentration utilisée pour le contrôle de seuil).

### EXEMPLES

### A. Immunodot pour la détection des anticorps anti-pyruvate déshydrogénase (M2).

Immunodot à 3 zones membranaires : 1 contrôle de réaction, 1 contrôle de seuil, 1 zone d'essai.
- Solution de dilution des ligands : NaCl 0,15 M dans H₂O distillée
- Ligand spécifique : Pyruvate déshydrogénase (SIGMA P-7032) à 0,25 mg/ml.
- Ligand non spécifique de contrôle de seuil : immunoglobuline de chèvre anti-molécule entière d'IgG humaine (SIGMA I-1886) à 0,017 mg/ml.
- Ligand non spécifique de contrôle de réaction : immunoglobuline de chèvre anti-molécule entière d'IgG humaine (SIGMA I-1886) à 1 mg/ml.

On dépose 1 µl de chacun des ligands sur la zone membranaire correspondante, puis on laisse sécher 24 heures à température ambiante.

### B. Immunodot pour la détection des anticorps antinucléaires (ENA).

Immunodot à 8 zones membranaires : 1 contrôle de réaction, 1 contrôle de seuil, 6 zones d'essai pour les anticorps anti-ENA suivants : Sm, RNP, SSA, SSB, JO-1, Scl-70.
- Solution de dilution des ligands : NaCl 0,15 M dans H₂O distillée.
- Ligands spécifiques : Sm antigène (IMMUNOVISION SMA-3000) à 0,16 mg/ml, RNP antigène (IMMUNOVISION SRC-3000) à 0,19 mg/ml, SSA antigène (IMMUNOVISION SSA-3000) à 0,15 mg/ml, SSB antigène (IMMUNOVISION SSB-3000) à 0,08 mg/ml, JO-1 antigène (IMMUNOVISION JO1-3000) à 0,07 mg/ml, Scl-70 antigène (IMMUNOVISION SCL-3000) à 0,07 mg/ml.
- Ligand de contrôle de seuil : immunoglobuline de chèvre anti-molécule entière d'IgG humaine (SIGMA I-1886) à 0,022 mg/ml.
- Ligand de contrôle de réaction : immunoglobuline de chèvre anti-molécule entière d'IgG humaine (SIGMA I-1886) à 1 mg/ml.

On dépose 1 µl de chacun des ligands sur la zone membranaire correspondante, puis on laisse sécher 24 heures à température ambiante.

### C. Immunodot pour la détection des anticorps anti-ADN double brin

Immunodot à 3 zones membranaires : 1 contrôle de réaction, 1 contrôle de seuil, 1 zone d'essai.
- Solution de dilution des ligands : NaCl 0,15 M, Tris-HCl 20 mM, pH 7,5.
- Ligand spécifique : ADN double brin extrait de thymus de veau (SIGMA D4522) à 0,2 mg/ml.
- Ligands non spécifiques de contrôle de seuil (les concentrations indiquées correspondent aux concentrations finales dans le mélange) : mélange de ADN simple brin (SIGMA D8899) à 10 µg/ml, Histone H1 (IMMUNOVISION HIS-1011) à 2,5 µg/ml, Histone H2a (IMMUNOVISION HIS-1002) à 2,5 µg/ml, Histone H2b (IMMUNOVISION HIS-1013) à 2,5 µg/ml, Histones H3/H4 (IMMUNOVISION HIS-1014) à 2,5 µg/ ml, BSA à 5 µg/ml.
- Ligands non spécifiques de contrôle de réaction (les concentrations indiquées correspondent aux concentrations finales dans le mélange) : mélange d'Immunoglobuline de chèvre anti-molécule entière d'IgG humaine (SIGMA I-1886) à 0,5 mg/ml et d'Immunoglobuline de lapin anti-molécule (µ-chain) d'IgM humaine (SIGMA I-0140) à 0,5 mg/ml.

On dépose 1 µl de chacun des ligands ou mélanges de ligands sur la zone membranaire correspondante, puis on laisse sécher 24 heures à température ambiante.

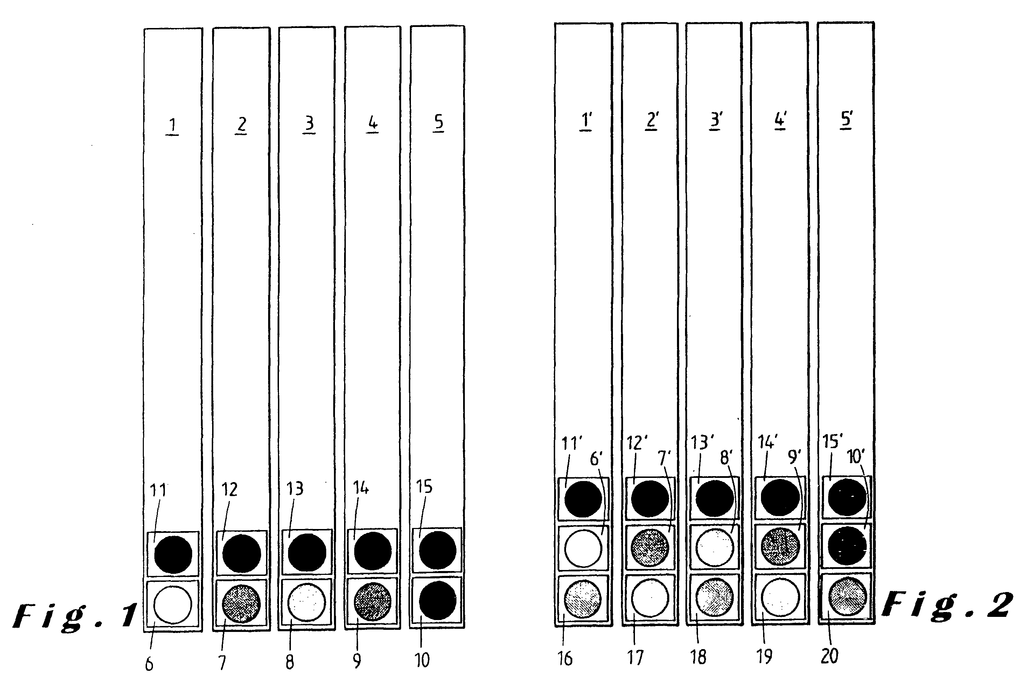

Les figures 1 et 2 annexées illustrent une comparaison d'interprétation des résultats obtenus par un procédé classique de dépistage et par le procédé suivant l'invention.

La figure 1 montre cinq bandelettes 1 à 5 courantes dans le commerce sur lesquelles ont été appliquées des gouttes d'un ligand spécifique pour un analyte déterminé dans les zones d'essai 6 à 10. Ces bandelettes sont aussi chacune pourvues d'une zone de contrôle de réaction 11 à 15 où un ligand non spécifique est appliqué à une concentration élevée permettant de contrôler si la réaction a lieu de manière conforme.

Cinq échantillons sont alors analysés quant à l'analyte à détecter suivant une procédure connue en soi, non décrite ici en détail. A la fin de l'essai immunodot une intensité de signal est déterminée dans chaque zone. Dans cet essai il s'agit d'un signal visuel, mais il pourrait s'agir d'une méthode permettant de fournir un signal à mesurer par radiométrie, fluorescence, luminescence, etc... Comme on peut le constater, les cinq zones de contrôle de réaction 11 à 15 donnent un signal fortement intense, les essais sont donc valables. Il faut noter que les intensités de signal de ces zones 11 à 15 sont légèrement variables entre elles d'une manière impossible à représenter sur le dessin, mais perceptible à l'oeil nu.

La zone 6 permet de déceler clairement que l'échantillon est négatif et les échantillons correspondant aux autres zones d'essai 7 à 10 seront probablement déterminés comme positifs par le laborantin.

La figure 2 montre cinq bandelettes 1' à 5' suivant l'invention sur lesquelles se trouvent des zones d'essais 6' à 10' et des zones de contrôle de réaction 11' à 15' qui sont identiques aux zones 6 à 15 des bandelettes 1 à 5 illustrées sur la figure 1 et qui donc donnent la même intensité de signal.

Ces bandelettes 1' à 5' présentent en outre chacune une zone de contrôle de seuil 16 à 20 qui ont été réalisées comme décrit ci-dessus. Les intensités de signal de ces zones sont légèrement variables d'une bandelette à l'autre, ce qui montre que chaque échantillon à analyser présente un potentiel de bruit de fond qui lui est propre.

Il ressort de l'essai illustré, que l'échantillon correspondant à la bandelette 3' est à considérer, d'une manière inattendue, comme négatif. Son intensité de signal est en effet clairement inférieure à celle de la zone de contrôle de seuil. La conclusion que l'on peut tirer de l'essai était imprévisible pour l'homme de métier faisant usage du procédé de dépistage selon la figure 1.

## Revendications

1. Procédé de dépistage d'au moins un analyte, dans un échantillon de fluide biologique à analyser, comprenant :
- une mise en contact de l'échantillon de fluide biologique avec un ligand, qui est spécifique d'au moins un analyte à dépister et qui est fixé sur au moins une zone d'essai d'un support solide,
- une liaison entre ledit au moins un analyte à dépister et le ligand,
- un marquage du ligand spécifique et dudit au moins un analyte liés, par un traceur, et
- une détection du traceur de marquage sur le ligand spécifique et ledit au moins un analyte liés, avec détermination d'une intensité de signal de marquage,
- une mise en contact de l'échantillon de fluide biologique avec un ligand qui est non spécifique dudit au moins un analyte, est fixé sur une zone de contrôle du support solide et est capable de rendre compte d'interactions non spécifiques engendrées par l'échantillon,
- une adsorption et/ou un accrochage de facteurs d'interactions non spécifiques de l'échantillon sur le ligand non spécifique,
- un marquage du ligand non spécifique et des facteurs d'interactions liés, par un traceur,
- une détection du traceur de marquage sur le ligand non spécifique et les facteurs d'interactions liés, avec détermination d'une intensité de signal de marquage,
**caractérisé en ce que** :
au moins un ligand non spécifique est fixé sur une zone de contrôle de seuil, en une concentration telle que l'intensité de signal de marquage déterminée là pour un échantillon seuil de fluide biologique est égale à celle de cet échantillon seuil sur la zone d'essai,
ledit au moins un ligand non spécifique de la zone de contrôle de seuil étant capable de rendre compte d'un potentiel de bruit de fond engendré sur au moins une zone d'essai par un ensemble de facteurs d'interactions non spécifiques, liés à la nature de l'échantillon lui-même et aux conditions dans lesquelles le test est effectué,
et **en ce que** le procédé comprend une comparaison entre l'intensité du signal de marquage de la zone d'essai et celle de la zone de contrôle de seuil obtenues avec l'échantillon à analyser, celui-ci étant à considérer comme positif lorsque l'intensité du signal de marquage de la zone d'essai est supérieure à celle de la zone de contrôle de seuil.

2. Procédé de dépistage d'analyte suivant la revendication 1, **caractérisé en ce qu'**il comprend
- une mise en contact de l'échantillon de fluide biologique à analyser avec au moins un ligand qui est non spécifique dudit au moins un analyte et qui est fixé sur une zone de contrôle dite de réaction du support solide,
- une adsorption et/ou un accrochage de facteurs d'interactions non spécifiques de l'échantillon sur ledit au moins un ligand non spécifique de la zone de contrôle de réaction,
- un marquage dudit au moins un ligand non spécifique de la zone de contrôle de réaction et des facteurs d'interaction liés, par un traceur,
- une détection du traceur de marquage sur ledit au moins un ligand non spécifique de la zone de contrôle de réaction et les facteurs d'interaction liés, avec détermination d'une intensité de signal de marquage,
- ledit au moins un ligand non spécifique de la zone de contrôle de réaction étant fixé sur celle-ci de manière que l'intensité de signal de marquage déterminée soit détectable lorsque le procédé de dépistage a été appliqué d'une manière conforme.

3. Procédé de dépistage suivant l'une ou l'autre des revendications 1 et 2, **caractérisé en ce qu'**il comprend
- une mise en contact de l'échantillon de fluide biologique avec au moins un ligand supplémentaire, qui est spécifique d'au moins un analyte supplémentaire à dépister et qui est fixé sur au moins une zone d'essai supplémentaire du support solide,
- une liaison entre ledit au moins un analyte supplémentaire et son ligand spécifique.
- un marquage dudit au moins un ligand supplémentaire et dudit au moins un analyte supplémentaire liés, par un traceur, et
- une détection du traceur de marquage sur ledit au moins un ligand supplémentaire et ledit au moins un analyte supplémentaire liés, avec détermination d'une intensité de signal de marquage,
- chaque ligand spécifique supplémentaire étant fixé dans sa zone d'essai supplémentaire en une concentration telle que l'intensité de signal de marquage déterminée là pour un échantillon seuil pour l'analyte supplémentaire correspondant est égale ou inférieure à l'intensité de signal de marquage obtenue dans ladite zone de contrôle de seuil, et
- une comparaison entre l'intensité du signal de marquage de chaque zone d'essai supplémentaire et celle de la zone de contrôle de seuil, obtenues avec l'échantillon à analyser, celui-ci étant à considérer comme positif pour un analyte supplémentaire lorsque l'intensité du signal de marquage de la zone d'essai supplémentaire correspondant à cet analyte supplémentaire est supérieure à celle de la zone de contrôle de seuil.

4. Procédé suivant l'une des revendications 2 et 3, **caractérisé en ce que** ledit au moins un ligand non spécifique fixé sur la zone de contrôle de seuil est ledit au moins un ligand non spécifique fixé sur la zone de contrôle de réaction.

5. Procédé suivant l'une quelconque des revendications 1 à 4, **caractérisé en ce que** ledit au moins un ligand non spécifique fixé sur la zone de contrôle de seuil est choisi parmi le groupe comprenant des anticorps, monoclonaux ou non, dirigés ou non contre un ou plusieurs antigènes contenus dans l'échantillon, des antigènes dirigés ou non contre un ou plusieurs anticorps contenus dans l'échantillon, des protéines synthétiques ou naturelles, telles que de l'albumine de sérum de boeuf, des protéines de lait, des séquences synthétiques ou naturelles d'acides aminés, comme un peptide synthétique, un haptène, de la polylysine, et des séquences nucléotidiques synthétiques ou naturelles, comme de l'ADN (simple brin ou double brin), de l'ARN.

6. Procédé suivant l'une quelconque des revendications 1 à 5, **caractérisé en ce que** la détection est effectuée par voie colorimétrique, enzymatique, radiométrique, luminescente.

7. Procédé suivant l'une quelconque des revendications 1 à 6, **caractérisé en ce que** ledit au moins un ligand non spécifique de la zone de contrôle de seuil est susceptible d'établir une liaison non spécifique avec l'analyte à dépister et rend compte, dans ledit potentiel de bruit de fond, d'interactions non spécifiques entre l'analyte à dépister et ledit au moins un ligand non spécifique.

8. Trousse pour le dépistage d'au moins un analyte dans un échantillon de fluide biologique à analyser, comprenant :
- un support solide,
- un ligand qui est spécifique d'au moins un analyte à dépister et est fixé sur au moins une zone d'essai du support solide, et qui, après mise en contact avec l'échantillon à analyser, se lie à ledit au moins un analyte à dépister, en présence de celui-ci,
- un traceur capable de marquer le ligand spécifique et ledit au moins un analyte liés, en permettant sa détection avec détermination d'une intensité de signal de marquage,
- un ligand qui est non spécifique dudit au moins un analyte et est fixé sur une zone de contrôle du support solide, et qui, après mise en contact avec l'échantillon à analyser, permet une adsorption et/ou un accrochage sur lui de facteurs d'interactions non spécifiques engendrés par l'échantillon, et
- un traceur capable de marquer le ligand non spécifique et les facteurs d'interactions liés, en permettant sa détection avec détermination d'une intensité de signal de marquage,
**caractérisé en ce qu'**elle comprend au moins un ligand non spécifique fixé sur une zone de contrôle de seuil du support solide en une concentration telle que l'intensité de signal de marquage, déterminée là pour un échantillon seuil de fluide biologique est égale à celle de cet échantillon seuil sur la zone d'essai,
l'échantillon à analyser étant à considérer comme positif lorsque l'intensité du signal de marquage de la zone d'essai est supérieure à celle de la zone de contrôle de seuil.

9. Trousse suivant la revendication 8, **caractérisée en ce qu'**elle comprend en outre
- au moins un ligand qui n'est pas spécifique dudit au moins un analyte à dépister et est fixé sur une zone de contrôle de réaction du support solide, et qui, après mise en contact avec l'échantillon à analyser, permet une adsorption et/ou un accrochage sur lui de facteurs d'interactions non spécifiques engendrés par l'échantillon,
- un traceur capable de marquer le ligand non spécifique et les facteurs d'interactions liés, en permettant sa détection avec détermination d'une intensité de signal de marquage,
- ledit au moins un ligand non spécifique étant fixé sur la zone de contrôle de réaction de manière que l'intensité de signal de marquage déterminée est décelable lorsque le procédé de dépistage a été appliqué d'une manière conforme.

10. Trousse suivant l'une ou l'autre des revendications 8 et 9, **caractérisée en ce qu'**elle comprend en outre
- au moins un ligand supplémentaire, qui est spécifique d'au moins un analyte supplémentaire à dépister et est fixé sur au moins une zone d'essai supplémentaire du support solide, et qui, après mise en contact avec l'échantillon à analyser, se lie à ledit au moins un analyte supplémentaire, en présence de celui-ci,
- un traceur capable de marquer ledit au moins un ligand supplémentaire spécifique et ledit au moins un analyte supplémentaire liés, en permettant sa détection avec détermination d'une intensité de signal de marquage,
- chaque ligand spécifique supplémentaire étant fixé dans sa zone d'essai supplémentaire en une concentration telle que l'intensité de signal de marquage, déterminée là pour un échantillon seuil pour l'analyte supplémentaire correspondant, est égale ou inférieure à l'intensité de signal de marquage obtenue dans ladite zone de contrôle de seuil,
- l'échantillon à analyser étant à considérer comme positif lorsque l'intensité du signal de marquage de la zone d'essai supplémentaire correspondant à cet analyte supplémentaire est supérieure à celle de la zone de contrôle de seuil.

## Patentansprüche

1. Verfahren zum Nachweis mindestens eines Analyten in einer Probe zu analysierender biologischer Flüssigkeit, umfassend:
- Inkontaktbringen der Probe biologischer Flüssigkeit mit einem Liganden, welcher spezifisch ist für mindestens einen nachzuweisenden Analyten und welcher fixiert ist auf mindestens einem Testbereich eines festen Trägers,
- eine Bindung zwischen dem mindestens einen nachzuweisenden Analyten und dem Liganden,
- eine Markierung des spezifischen Liganden und des mindestens einen gebundenen Analyten durch einen Tracer und
- einen Nachweis des Markierungs-Tracers auf dem spezifischen Liganden und dem mindestens einen gebundenen Analyten durch Bestimmung einer Intensität des Markierungssignals,
- Inkontaktbringen der Probe biologischer Flüssigkeit mit einem Liganden, welcher unspezifisch ist für den mindestens einen Analyten, welcher auf einem Kontrollbereich des festen Trägers fixiert ist und in der Lage ist, unspezifischen Interaktionen, verursacht durch die Probe, Rechnung zu tragen,
- eine Adsorption und/oder eine Bindung von unspezifischen Interaktionsfaktoren der Probe auf dem unspezifischen Liganden,
- eine Markierung des unspezifischen Liganden und gebundener Interaktionsfaktoren mit einem Tracer,
- einen Nachweis des Markierungs-Tracers auf dem unspezifischen Liganden und der gebundenen Interaktionsfaktoren durch Bestimmung einer Intensität des Markierungssignals, **dadurch gekennzeichnet, dass**:
mindestens ein unspezifischer Ligand fixiert ist auf einem Kontrollbereich eines Schwellenwertes mit einer Konzentration dergestalt, dass die Intensität des Markierungssignals, welches dort in einer Schwellenwert-Probe aus biologischer Flüssigkeit festgestellt wurde, der Intensität dieser Schwellenwert-Probe auf dem Testbereich entspricht,
wobei der mindestens eine unspezifische Ligand des Schwellenwert-Kontrollbereichs in der Lage ist, einem Potential an Grundrauschen Rechnung zu tragen, erzeugt auf mindestens einem Testbereich durch eine Gesamtheit von unspezifischen Interaktionsfaktoren, welche zusammenhängen mit der Natur der Probe selbst und mit den Bedingungen, unter welchen der Test ausgeführt wird,
und **dadurch gekennzeichnet, dass** das Verfahren einen Vergleich umfasst zwischen der Markierungssignalintensität des Testbereichs und des Schwellenwert-Kontrollbereichs, welche erhalten werden durch die zu analysierende Probe, wobei die Probe als positiv zu erachten ist, wenn die Intensität des Markierungssignals des Versuchsbereichs größer ist verglichen mit der Intensität des Schwellenwert-Kontrollbereichs.

2. Verfahren zum Nachweis eines Analyten nach Anspruch 1, umfassend
- Inkontaktbringen der Probe zu analysierender biologischer Flüssigkeit mit mindestens einem Liganden, welcher unspezifisch gegenüber dem mindestens einen Analyten ist und welcher fixiert ist auf einem Kontrollreaktionsbereich des festen Trägers,
- eine Adsorption und/oder eine Bindung von unspezifischen Interaktionsfaktoren der Probe auf dem mindestens einen unspezifischen Liganden des Kontrollreaktionsbereichs,
- eine Markierung des mindestens einen unspezifischen Liganden des Kontrollreaktionsbereichs und der gebundenen Interaktionsfaktoren mit einem Tracer,
- einen Nachweis des Markierungs-Tracers auf dem mindestens einen unspezifischen Liganden des Kontrollreaktionsbereichs und der gebundenen Interaktionsfaktoren durch Bestimmung einer Markierungssignalintensität,
- wobei der mindestens eine unspezifische Ligand des Kontrollreaktionsbereichs dergestalt auf diesem fixiert ist, dass die Intensität des bestimmten Markierungssignals nachweisbar ist, wenn das Nachweisverfahren unter Standardbedingungen angewandt wurde.

3. Nachweisverfahren nach Anspruch 1 oder 2, umfassend
- Inkontaktbringen der Probe biologischer Flüssigkeit mit mindestens einem weiteren Liganden, welcher spezifisch ist für mindestens einen weiteren nachzuweisenden Analyten und welcher fixiert ist auf mindestens einem weiteren Testbereich des festen Trägers,
- eine Bindung zwischen dem mindestens einen weiteren Analyten und seinem spezifischen Liganden,
- eine Markierung des mindestens einen weiteren Liganden und des mindestens einen weiteren gebundenen Analyten mittels eines Tracers und
- einen Nachweis des Markierungs-Tracers auf dem mindestens einen weiteren Liganden und dem mindestens einen weiteren gebundenen Analyten durch Bestimmung einer Markierungssignalintensität,
- wobei jeder weitere spezifische Ligand in seinem weiteren Testbereich in einer Konzentration dergestalt fixiert ist, dass die Intensität des Markierungssignals, welche dort für eine Schwellenwertprobe für den korrespondierenden weiteren Analyten festgestellt wurde, gleich oder geringer ist verglichen mit der Markierungssignalintensität, welche in dem Schwellenwert-Kontrollbereich erhalten wurde, und
- einen Vergleich zwischen der Markierungssignalintensität jedes weiteren Testbereiches und des Bereiches der Schwellenwert-Kontrolle, welche erhalten wurden durch die zu analysierende Probe, wobei die Probe als positiv zu erachten ist für einen weiteren Analyten, wenn die Markierungssignalintensität des weiteren Testbereichs, welcher diesem weiteren Analyten entspricht, größer ist als die des Schwellenwert-Kontrollbereichs.

4. Verfahren nach Anspruch 2 oder 3, **dadurch gekennzeichnet, dass** der mindestens eine unspezifische Ligand, welcher auf dem Schwellenwert-Kontrollbereich fixiert ist, der mindestens eine unspezifische Ligand ist, welcher auf dem Kontrollreaktionsbereich fixiert ist.

5. Verfahren nach einem der Ansprüche 1 bis 4, **dadurch gekennzeichnet, dass** der mindestens eine unspezifische Ligand, welcher auf dem Schwellenwert-Kontrollbereich fixiert ist, ausgewählt ist aus der Gruppe bestehend aus monoclonalen oder nicht monoclonalen Antikörpern, welche gegen eines oder mehrere Antigene, welche in der Probe enthalten sind, gerichtet sind oder nicht, Antigenen, die gegen einen oder mehrere Antikörper, welche in der Probe enthalten sind, gerichtet sind oder nicht, synthetischen oder natürlichen Proteinen, wie z.B. Rinderserumalbumin, Milchproteinen, synthetischen oder natürlichen Aminosäuresequenzen, wie ein synthetisches Peptid, ein Hapten, Polylysin, und synthetischen oder natürlichen Nucleotidsequenzen, wie DNA (einzelsträngig oder doppelsträngig) und RNA.

6. Verfahren nach einem der Ansprüche 1 bis 5, **dadurch gekennzeichnet, dass** der Nachweis auf kalorimetrischem, enzymatischem, radiometrischem, luminiszentem Wege ausgeführt wird.

7. Verfahren nach einem der Ansprüche 1 bis 6, **dadurch gekennzeichnet, dass** der mindestens eine unspezifische Ligand des Schwellenwert-Kontrollbereichs in der Lage ist, eine unspezifische Bindung einzugehen mit dem nachzuweisenden Analyten, und den unspezifischen Interaktionen zwischen dem nachzuweisenden Analyten und dem mindestens einen unspezifischen Liganden in dem Potential an Grundrauschen Rechnung trägt.

8. Kit zum Nachweis mindestens eines Analyten in einer Probe zu analysierender biologischer Flüssigkeit, umfassend:
- einen festen Träger,
- einen Liganden, welcher spezifisch für mindestens einen nachzuweisenden Analyten ist, und welcher fixiert ist auf mindestens einem Testbereich des festen Trägers, und welcher nach Inkontaktbringen mit der zu analysierenden Probe an den mindestens einen nachzuweisenden Analyten bindet, in Anwesenheit desselben,
- einen Tracer, welcher in der Lage ist, den spezifischen Liganden und den mindestens einen gebundenen Analyten zu markieren, indem er seinen Nachweis durch Bestimmung einer Markierungssignalintensität ermöglicht,
- einen Liganden, welcher unspezifisch ist gegenüber dem mindestens einen Analyten und welcher fixiert ist auf einem Kontrollbereich des festen Trägers, und welcher nach Inkontaktbringen mit der zu analysierenden Probe an ihm eine Adsorption und/oder eine Bindung unspezifischer Interaktionsfaktoren ermöglicht, welche durch die Probe verursacht sind, und
- einen Tracer, welcher in der Lage ist, den unspezifischen Liganden und die gebundenen Interaktionsfaktoren zu markieren, indem sein Nachweis durch Bestimmung einer Markierungssignalintensität ermöglicht wird,
**dadurch gekennzeichnet, dass** er mindestens einen unspezifischen Liganden umfasst, welcher fixiert ist auf einem Schwellenwert-Kontrollbereich des festen Trägers in einer Konzentration dergestalt, dass die Intensität des Markierungssignals, welches dort bestimmt wird mit Hilfe einer Schwellenwertprobe von biologischer Flüssigkeit, der Intensität dieser Schwellenwertprobe auf dem Testbereich entspricht,
wobei die zu analysierende Probe als positiv zu erachten ist, wenn die Markierungssignalintensität des Testbereichs größer ist als diejenige des Schwellenwert-Kontrollbereichs.

9. Kit nach Anspruch 8, **dadurch gekennzeichnet, dass** er unter anderem umfasst
- mindestens einen Liganden, welcher nicht spezifisch ist gegenüber dem mindestens einen nachzuweisenden Analyten und welcher fixiert ist auf einem Kontrollreaktionsbereich des festen Trägers, und welcher, nach Inkontaktbringen mit der zu analysierenden Probe, an ihm eine Adsorption und/oder eine Bindung unspezifischer Interaktionsfaktoren ermöglicht, welche durch die Probe verursacht sind,
- einen Tracer, welcher in der Lage ist, den unspezifischen Liganden und die gebundenen Interaktionsfaktoren zu markieren, indem er ermöglicht, durch Bestimmung einer Markierungssignalintensität nachgewiesen zu werden,
- wobei der mindestens eine unspezifische Ligand auf dem Kontrollreaktionsbereich dergestalt fixiert ist, dass die bestimmte Markierungssignalintensität nachweisbar ist, wenn das Nachweisverfahren unter Standardbedingungen angewandt wurde.

10. Kit nach Anspruch 8 oder 9, **dadurch gekennzeichnet, dass** er unter anderem umfasst
- mindestens einen weiteren Liganden, welcher spezifisch für mindestens einen weiteren nachzuweisenden Analyten ist und welcher fixiert ist auf mindestens einem weiteren Nachweisbereich des festen Trägers, und welcher nach Inkontaktbringen mit der zu analysierenden Probe an den mindestens einen weiteren Analyten bindet, in Anwesenheit desselben,
- einen Tracer, welcher in der Lage ist, den mindestens einen weiteren spezifischen Liganden und den mindestens einen weiteren gebundenen Analyten zu markieren, indem er seinen Nachweis mit Hilfe der Bestimmung einer Markierungssignalintensität ermöglicht,
- wobei jeder weitere spezifische Ligand in seinem weiteren Testbereich in einer Konzentration fixiert ist dergestalt, dass die Markierungssignalintensität, welche dort für eine Schwellenwertprobe für den weiteren korrespondierenden Analyten bestimmt wird, gleich oder geringer ist als die Markierungssignalintensität, welche in dem Schwellenwert-Kontrollbereich erhalten wurde,
- und wobei die zu analysierende Probe als positiv zu erachten ist, falls die Markierungssignalintensität des weiteren Kontrollbereichs, welcher diesem weiteren Analyten entspricht, größer ist als diejenige des Schwellenwert-Kontrollbereichs.

## Claims

1. Method of detecting at least one analyte, in a sample of biological fluid to be analysed, comprising:
- putting the sample of biological fluid into contact with a ligand which is specific to at least one analyte to be detected and which is fixed to at least one test area of a firm support,
- connection between the said at least one analyte to be detected and the ligand,
- marking of the specific ligand and the said at least one analyte connected, by means of a tracer, and
- detection of the marking tracer on the specific ligand and the said at least one analyte connected, with determination of a marking signal intensity,
- putting the sample of biological fluid into contact with the ligand which is non-specific to the said at least one analyte, is fixed to a control area of the firm support and is capable of taking account of non-specific interactions generated by the sample,
- adsorption and/or attachment of interaction factors not specific to the sample on the non-specific ligand,
- marking of the non-specific ligand and interaction factors connected, by means of a tracer,
- detection of the marking tracer on the non-specific ligand and the interaction factors connected, with determination of a marking signal intensity,
**characterised in that**:
at least one non-specific ligand is fixed to a threshold control area, in a concentration such that the marking signal intensity determined there for a threshold sample of biological fluid is equal to that of this threshold sample on the test area,
the said at least one non-specific ligand of the threshold control area being capable of taking account of a background noise potential generated on at least one test area by a set of non-specific interaction factors, related to the nature of the sample itself and to the conditions in which the test is carried out,
and **in that** the method comprises a comparison between the intensity of the test area marking signal and that of the threshold control area obtained with the sample to be analysed, this being considered to be positive when the intensity of the test area marking signal is greater than that of the threshold control area.

2. Analyte detection method according to Claim 1, **characterised in that** it comprises
- putting the sample of biological fluid to be analysed in contact with at least one ligand which is non-specific to the said at least one analyte and which is fixed to a so-called reaction control area of the solid support,
- adsorption and/or attachment of interaction factors not specific to the sample on the said at least one ligand non-specific to the reaction control area,
- marking of the said at least one ligand not specific to the reaction control area and the interaction factors connected, by means of a tracer,
- detection of the marking tracer on the said at least one ligand not specific to the reaction control area and the interaction factors connected, with determination of a marking signal intensity,
- the said at least one ligand non-specific to the reaction control area being fixed to the latter so that the marking signal intensity determined is detectable when the detection method has been applied in a conforming manner.

3. Detection method according to one or other of Claims 1 and 2, **characterised in that** it comprises
- putting the sample of biological fluid in contact with at least one supplementary ligand, which is specific to at least one supplementary analyte to be detected and which is fixed to at least one supplementary test area of the firm support,
- connection between the said at least one supplementary analyte and its specific ligand,
- marking of the said at least one supplementary ligand and the said at least one supplementary analyte connected, by means of a tracer, and
- a detection of the marking tracer on the said at least one supplementary ligand and the said at least one supplementary analyte connected, with determination of a marking signal intensity,
- each supplementary specific ligand being fixed in its supplementary test area in a concentration such that the marking signal intensity determined there for a threshold sample for the corresponding supplementary analyte is equal to or less than the marking signal intensity obtained in the said threshold control area, and
- comparison between the intensity of the marking signal of each supplementary test area and that of the threshold control area, obtained with the sample to be analysed, this being to be considered to be positive for a supplementary analyte when the intensity of the marking signal of the supplementary test area corresponding to this supplementary analyte is greater than that of the threshold control area.

4. Method according to one of Claims 2 and 3, **characterised in that** the said at least one non-specific ligand fixed to the threshold control area is the said at least one non-specific ligand fixed to the reaction control area.

5. Method according to any one of Claims 1 to 4, **characterised in that** the said at least one non-specific ligand fixed to the threshold control area is chosen from amongst the group comprising antibodies, monoclonal or not, directed or not against one or more antigens contained in the sample, antigens directed or not against one or more antibodies contained in the sample, synthetic or natural proteins, such as bovine serum albumin, milk proteins, synthetic or natural sequences of amino acids, such as a synthetic peptide, a haptene, polylysine, and synthetic or natural nucleotide sequences, such as DNA (single strand or double strand) or RNA.

6. Method according to any one of Claims 1 to 5, **characterised in that** the detection is made by colorimetric, enzymatic, radiometric or luminescent method.

7. Method according to any one of Claims 1 to 6, **characterised in that** the said at least one ligand non-specific to the threshold control area is able to establish a non-specific connection with the analyte to be detected and takes account, in the said background noise potential, of non-specific interactions between the analyte to be detected and the said at least one non-specific ligand.

8. Kit for detecting at least one analyte in a sample of biological fluid to be analysed, comprising:
- a firm support,
- a ligand which is specific to at least one analyte to be detected and is fixed to at least one test area of the firm support and which, after putting into contact with the sample to be analysed, connects to the said at least one analyte to be detected, in the presence thereof,
- a tracer capable of marking the specific ligand and the said at least one analyte connected, allowing its detection with determination of a marking signal intensity,
- a ligand which is non-specific to the said at least one analyte and is fixed to a control area of the firm support and which, after putting into contact with the sample to be analysed, allows adsorption and/or attachment to it of non-specific interaction factors generated by the sample, and
- a tracer capable of marking the non-specific ligand and the interaction factors connected, allowing its detection with determination of a marking signal intensity,
**characterised in that** it comprises at least one non-specific ligand fixed to a threshold control area of the firm support in a concentration such that the marking signal intensity, determined there for a biological fluid threshold sample, is equal to that of this threshold sample on the test area,
the sample to be analysed being considered to be positive when the intensity of the marking signal of the test area is greater than that of the threshold control area.

9. Kit according to Claim 8, **characterised in that** it also comprises
- at least one ligand which is not specific to the said at least one analyte to be detected and is fixed to a reaction control area of the firm support and which, after putting into contact with the sample to be analysed, allows adsorption and/or attachment to it of non-specific interaction factors generated by the sample,
- a tracer capable of marking the non-specific ligand and the interaction factors connected, allowing its detection with the determination of a marking signal intensity,
- the said at least one non-specific ligand being fixed to the reaction control area so that the marking signal intensity determined is discernable when the detection method has been applied in a conforming manner.

10. Kit according to one or other of Claims 8 and 9, **characterised in that** it also comprises
- at least one supplementary ligand, which is specific to at least one supplementary analyte to be detected and is fixed to at least one supplementary test area of the firm support and which, after putting into contact with the sample to be analysed, connects to the said at least one supplementary analyte, in the presence thereof,
- a tracer capable of marking the said at least one specific supplementary ligand and the said at least one supplementary analyte connected, allowing its detection with determination of a marking signal intensity,
- each supplementary specific ligand being fixed in its supplementary test area in a concentration such that the marking signal intensity, determined there for a threshold sample for the corresponding supplementary analyte, is equal to or less than the marking signal intensity obtained in the said threshold control area,
- the sample to be analysed being to be considered as positive when the intensity of the marking signal of the supplementary test area corresponding to this supplementary analyte is greater than that of the threshold control area.
